(19) Europäisches Patentamt
European Patent Office
Office européen des brevets

(11) EP 3 210 593 B1

(12) **EUROPEAN PATENT SPECIFICATION**

(45) Date of publication and mention of the grant of the patent:
**30.09.2020 Bulletin 2020/40**

(51) Int Cl.:
***A61K 8/44*** *(2006.01)* ***A61Q 5/04*** *(2006.01)*
***A45D 7/04*** *(2006.01)*

(21) Application number: **17157778.6**

(22) Date of filing: **24.02.2017**

(54) **HAIR SHAPE CONTROL ASSISTING AGENT**

UNTERSTÜTZENDES MITTEL ZUR HAARFORMKONTROLLE

AGENT D'AIDE AU CONTRÔLE DE LA MISE EN FORME DES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.02.2016 JP 2016032680**

(43) Date of publication of application:
**30.08.2017 Bulletin 2017/35**

(73) Proprietor: **AJINOMOTO CO., INC.**
**Chuo-ku**
**Tokyo 104-8315 (JP)**

(72) Inventors:
- **KOBAYASHI, Shun**
**Kanagawa, 210-8681 (JP)**
- **TAKAHASHI, Kenta**
**Takasaki City, 370-1201 (JP)**

(74) Representative: **Mewburn Ellis LLP**
**Aurora Building**
**Counterslip**
**Bristol BS1 6BX (GB)**

(56) References cited:
**EP-A1- 1 473 027**
**WO-A1-2016/017659**
**WO-A1-2016/104694**
**JP-A- S5 679 618**
**JP-A- 2000 327 545**
**JP-A- 2004 323 505**
**JP-A- 2012 171 897**

- **SUZUKI M ET AL: "L-lysine based gemini organogelators: their organogelation properties and thermally stable organogels", ORGANIC & BIOMOLECULAR CHEMISTRY, ROYAL SOCIETY OF CHEMISTRY, GB, vol. 1, no. 22, 21 November 2003 (2003-11-21), pages 4124-4131, XP002276454, ISSN: 1477-0520, DOI: 10.1039/B308371C**
- **MASAHIRO SUZUKI ET AL: "Novel dumbbell-form low-molecular-weight gelators based on l-lysine: their hydrogelation and organogelation properties", NEW JOURNAL OF CHEMISTRY, vol. 29, no. 11, 1 January 2005 (2005-01-01), page 1439, XP55346030, GB ISSN: 1144-0546, DOI: 10.1039/b511158g**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[TECHNICAL FIELD OF THE INVENTION]

The present invention relates to an agent for assisting control of hair shape, which contains an acyl basic amino acid derivative, and a method of controlling the hair shape.

[BACKGROUND OF THE INVENTION]

When a permanent wave treatment or a curling treatment is applied, generally, a two-agent type method using a first agent containing reducing agents such as thioglycolic acid or a salt thereof, and cysteine or a salt thereof, and a second agent containing oxidizing agents such as sodium bromate, hydrogen peroxide and the like is performed to impart a desired wave shape or straight shape to the hair.

To be specific, the disulfide bond of keratin in the hair is reduced, that is, cleaved, by the reducing agent in the first agent to give a sulfhydryl group.

Then, generally, a desired wave shape or straight shape is formed and, while maintaining the desired shape, the cleaved disulfide bond is reformed at a new position by the oxidizing agent in the second agent to fix the shape.

However, when the hair is treated with the above-mentioned two-agent type hair treatment agent for permanent wave or curling treatment, the hair is sometimes damaged by the utilization of the reducing and oxidizing actions. Particularly, in the disulfide reforming process by the second agent, a part of the sulfhydryl group and a disulfide bond uncleaved by the first agent may be subject to excessive oxidation even up to a sulfonic acid group, in which case the sulfonic acid group once formed cannot be reduced by a reducing agent used for a permanent wave treatment and a curling treatment, and remains as it is.

When the disulfide bond in hair keratin decreases in this way, a desired shape cannot be easily formed with the hair and the imparted shape cannot be maintained with ease. Also, the hair components (matrix substances) start to elute out, as a result of which the appearance such as gathering of hair and the feeling of hair touch when a finger is run through it are lost.

In recent years, the frequency of coloring treatments accompanied by bleaching treatment and bleaching action has increased mainly in women, which in turn has produced severe damage to the hair. For the hair repeatedly subjected to such treatments, wave formation by permanent wave treatments and curling treatments is difficult, and the hair shows markedly degraded feeling of hair touch (softness, smoothness, gathering property, gloss etc.).

To solve such problems, the following permanent wave agents and curling agents have been proposed.

To further suppress hair damage by a permanent wave treatment, it has been proposed to use cysteamine as the first agent instead of reducing agents such as thioglycolic acid, cysteine and the like, and use cysteamine and an anionic surfactant in combination (patent document 1).

It has also been proposed to contain carboxymethyldextran sodium in the second agent, thereby improving gathering of wave and feeling of hair touch, and further, after the aforementioned treatment with the second agent, apply a treatment with a hair treatment agent containing a cationic compound (patent document 2).

Furthermore, it has also been proposed to add cysteamine and anion polymer to the first agent, and add, in addition to the oxidizing agent, a cationic surfactant comprising quaternary ammonium salt to the second agent (patent document 3) .

However, these techniques are not sufficiently satisfactory for maintaining the feeling of hair touch (softness, smoothness, gathering property, gloss etc.), suppressing hair damage, and maintaining a permanent wave effect.

[Document List]

[Patent documents]

patent document 1: JP-A-2007-01916
patent document 2: JP-A-2009-57335
patent document 3: JP-A-2013-95696

[SUMMARY OF THE INVENTION]

[Problems to be Solved by the Invention]

The present invention, as set out in the claims, provides an agent for assisting control of hair shape, which has effects of improving the feeling of hair touch after a permanent wave treatment or a curling treatment, and imparting and sustaining shape of the hair while suppressing damage thereto, and a method of treating hair, which is capable of exhibiting the above-mentioned effect by using the assisting agent.

[Means of Solving the Problems]

The present inventors have conducted intensive studies in an attempt to achieve the above-mentioned object, and found that a composition containing component (A): a compound represented by the following formula (1) (hereinafter to be also referred to as "compound (1)") or a salt thereof can markedly improve the feeling of hair touch after a permanent wave treatment or a curling treatment, as well as sustain shape of the hair while suppressing damage thereto, and maintain the effect thereof, which resulted in the completion of the present invention.

Therefore, the present invention provides the following.

[1] Use of an agent in a permanent wave treatment or a curling treatment of hair, the agent comprising component (A): a compound represented by the formula (1)

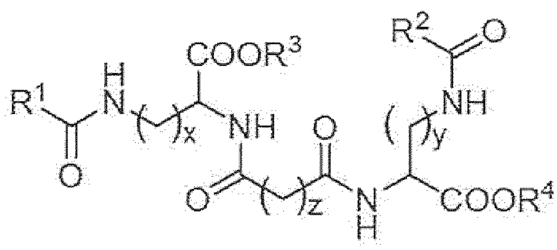

(1)

wherein

$R^1$ and $R^2$ are each independently an alkyl group having 5 - 21 carbon atoms or an alkenyl group having 5 - 21 carbon atoms,
$R^3$ and $R^4$ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof.

[2] The use according to [1], wherein the compound represented by the formula (1) is used for improving feeling of hair touch after a permanent wave treatment or curling treatment.
[3] The use according to [1], wherein the compound represented by the formula (1) is used for imparting and sustaining shape of the hair whilst suppressing hair damage.
[4] The use according to [1], wherein the compound represented by the formula (1) is used for assisting formation and holding of wave during hair wave shape adjustment.
[5] The use according to any one of [1] to [4], wherein the component (A) is a compound of the aforementioned formula (1) wherein z is an integer of 0 - 10 or a salt thereof.
[6] The use according to any one of [1] to [5], wherein the component (A) is a compound of the aforementioned formula (1) wherein z is 7 or 8 or a salt thereof.
[7] The use according to any one of [1] to [6], wherein the component (A) is a compound of the aforementioned formula (1) wherein x and y are each 4 or a salt thereof.
[8] The use according to any one of [1] to [7], wherein the component (A) is a compound of the aforementioned formula (1) wherein $R^1$ and $R^2$ are each independently a straight chain alkyl group having 5 - 15 carbon atoms or a salt thereof.
[9] The use according to any one of [1] to [8], wherein the component (A) is a compound of the aforementioned formula (1) wherein $R^3$ and $R^4$ are each a hydrogen atom or a salt thereof.
[10] The use according to any one of [1] to [9], wherein the component (A) is bis ($N^{\varepsilon}$-lauroyl-L-lysine)sebacoyl amide or a salt thereof.

[11] The use according to any one of [1] to [10], wherein the agent further comprises component (B): water in an amount of 10 - 10000 parts by weight per 1 part by weight of component (A).
[12] A first agent for a permanent wave treatment or a curling treatment of hair, comprising:

(i) a compound represented by the formula (1)

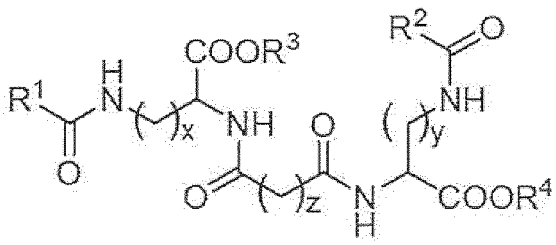

(1)

wherein

$R^1$ and $R^2$ are each independently an alkyl group having 5 - 21 carbon atoms or an alkenyl group having 5 - 21 carbon atoms,
$R^3$ and $R^4$ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof;

(ii) a reducing agent and
(iii) an alkali agent.

[13] A method of controlling the shape of hair, comprising the following:

(a) a step of applying a composition comprising component (A): a compound represented by the formula (1)

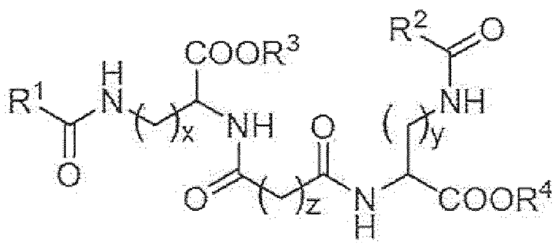

(1)

wherein

$R^1$ and $R^2$ are each independently an alkyl group having 5 - 21 carbon atoms or an alkenyl group having 5 - 21 carbon atoms,
$R^3$ and $R^4$ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof to hair,

(b) a step of applying a first agent containing a reducing agent, for a permanent wave treatment or a curling treatment to the hair,
(c) a step of applying a second agent containing an oxidizing agent, for a permanent wave treatment or a curling treatment to the hair.

[14] The method according to [13], wherein (a) and (b) are simultaneously performed; or
wherein (a), (b) and (c) are performed in this order; or
wherein (b), (a) and (c) are performed in this order.

[Effect of the Invention]

According to the present invention, the feeling of hair touch after a permanent wave treatment and a curling treatment can be improved and damage to the hair can be suppressed.
According to the present invention, the wave and the like after a permanent wave treatment and a curling treatment can be maintained for a long term.

[BRIEF DESCRIPTION OF THE DRAWINGS]

Fig. 1 shows the results of a hair tensile strength test. As reducing agents, ammonium thioglycolate and cysteamine were used (N=20).
Fig. 2 shows the results of waving efficiency and a wave maintenance rate test by the Kirby method. As a reducing agent, cysteamine was used (N=6).

[Description of Embodiments]

The hair shape control assisting agent of the present invention comprises component (A): a compound represented by the formula (1)

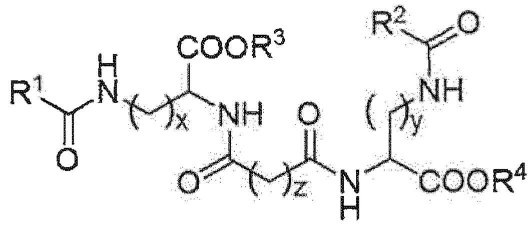 (1)

wherein

$R^1$ and $R^2$ are each independently an alkyl group having 5 - 21 carbon atoms or an alkenyl group having 5 - 21 carbon atoms,
$R^3$ and $R^4$ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof (hereinafter sometimes to be also referred to as the assisting agent of the present invention).

In the present invention, the "control of hair shape" means a "permanent wave treatment" and a "curling treatment", which are treatments for forming a wave, and the "permanent wave treatment" also includes a "hair straightening treatment". Specifically, the "permanent wave treatment" and the "curling treatment" mean treatments including cleaving the disulfide bond of the hair with the first agent and reforming the disulfide bond, cleaved by the second agent, at a new position to fix the desired shape.
The "hair shape control assisting agent" means a composition used for assisting formation and holding of wave during wave shape adjustment and assisting protection of hair. Specifically, it means a composition used as a first agent together with a reducing agent and an alkali agent, as a pretreatment agent to be applied to hair prior to the first agent, or as an intermediate agent to be applied to hair between the first agent and the second agent.
The mode of embodiment of the present invention is described in detail in the following.

1. component (A): a compound represented by the formula (1) (compound (1)) or a salt thereof

$R^1$ and $R^2$ are each independently an alkyl group having 5 - 21 carbon atoms or an alkenyl group having 5 - 21 carbon atoms.
The alkyl group having 5 - 21 carbon atoms means a straight chain or branched alkyl group having 5 - 21 carbon atoms, and specifically includes pentyl group, isopentyl group, neopentyl group, hexyl group, isohexyl group, neohexyl group, heptyl group, isoheptyl group, neoheptyl group, octyl group, isooctyl group, nonyl group, isononyl group, decyl

group, isodecyl group, undecyl group, dodecyl group, tridecyl group, tetradecyl group, pentadecyl group, hexadecyl group, heptadecyl group, octadecyl group, nonadecyl group, icosyl group and the like.

The alkenyl group having 5 - 21 carbon atoms means a straight chain or branched alkenyl group having 5 - 21 carbon atoms, and specifically includes pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

The alkyl group having 5 - 15 carbon atoms means a straight chain or branched alkyl group having 5 - 15 carbon atoms, and specifically includes pentyl group, hexyl group, heptyl group, octyl group, nonyl group, decyl group, undecyl group, dodecyl group, tridecyl group, tetradecyl group, pentadecyl group and the like.

The alkyl group having 7 - 11 carbon atoms means a straight chain or branched alkyl group having 7 - 11 carbon atoms, and specifically includes heptyl group, octyl group, nonyl group, decyl group, undecyl group and the like.

Preferably, $R^1$ and $R^2$ are each independently an alkyl group having 5 - 15 carbon atoms, more preferably they are each independently an alkyl group having 7 - 11 carbon atoms.

Preferably, $R^1$ and $R^2$ are each a straight chain alkyl group. Furthermore, $R^1$ and $R^2$ are preferably the same.

$R^3$ and $R^4$ are each independently a hydrogen atom, an alkyl group having 1 - 22 carbon atoms or an alkenyl group having 2 - 22 carbon atoms.

The alkyl group having 1 - 22 carbon atoms means a straight chain or branched alkyl group having 1 - 22 carbon atoms, and specifically includes methyl group, ethyl group, propyl group, isopropyl group, butyl group, isobutyl group, sec-butyl group, tert-butyl group, pentyl group, isopentyl group, neopentyl group, hexyl group, isohexyl group, neohexyl group, heptyl group, isoheptyl group, neoheptyl group, octyl group, isooctyl group, nonyl group, isononyl group, decyl group, isodecyl group, undecyl group, dodecyl group, tridecyl group, tetradecyl group, pentadecyl group, hexadecyl group, heptadecyl group, octadecyl group, nonadecyl group, icosyl group and the like.

The alkenyl group having 2 - 22 carbon atoms means a straight chain or branched alkenyl group having 2 - 22 carbon atoms, and specifically includes ethenyl group, 1-propenyl group, 2-propenyl group, 1-butenyl group, 2-butenyl group, 3-butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

$R^3$ and $R^4$ are preferably hydrogen atoms.

z is an integer of not less than 0.

z is preferably an integer of 0 - 10, more preferably 7 or 8.

x and y are each independently an integer of 2 - 4.

x and y are each preferably 4.

As the compound represented by the formula (1), the following compounds are preferable.

(compound A)

A compound wherein $R^1$ and $R^2$ are each independently a straight chain alkyl group having 5 - 15 carbon atoms,
$R^3$ and $R^4$ are each a hydrogen atom,
z is an integer of 0 - 10, and
x and y are each 4.

(compound B)

A compound wherein $R^1$ and $R^2$ are each a straight chain alkyl group having 5 - 15 carbon atoms,
$R^3$ and $R^4$ are each a hydrogen atom,
z is 7 or 8, and
x and y are each 4.

(compound C)

A compound wherein $R^1$ and $R^2$ are each a straight chain alkyl group having 7 - 11 carbon atoms,
$R^3$ and $R^4$ are each a hydrogen atom,
z is 7 or 8, and
x and y are each 4.

Specific examples of the compound represented by the formula (1) include

bis($N^{\varepsilon}$-lauroyl-L-lysine)sebacoyl amide, and
bis($N^{\varepsilon}$-octanoyl-L-lysine)sebacoyl amide,

and a salt thereof.

A salt of the compound represented by the formula (1) is not particularly limited and, for example, inorganic salts (e.g., alkali metal salts such as sodium salt, potassium salt and the like, alkaline earth metal salts such as calcium salt, magnesium salt and the like, aluminum salt, a salt with zinc and the like), and organic salts (e.g., organic amine salts such as ammonium salt, monoethanolamine salt, diethanolamine salt, triethanolamine salt and the like, basic amino acid salts such as arginine salt, lysine salt and the like, and the like). One kind of these may be used or two or more kinds selected from the above-mentioned group may be mixed and used. From the aspects of easy availability, handling property and the like, alkali metal salt, organic amine salt and basic amino acid salt are preferable, and sodium salt and potassium salt are particularly preferable.

Compound (1) is a known compound, and can be produced by a method known per se or a method analogous thereto (see JP-A-2004-323505, Org. Biomol. Chem., 2003, 1, 4124-4131, New J. Chem., 2005, 29, 1439-1444 and the like).

The content of component (A) in the assisting agent of the present invention is generally 0.001 - 10 wt%, preferably 0.005 - 5 wt%, more preferably 0.01 - 2 wt%.

2. component (B): water

The assisting agent of the present invention may contain water. Water in the present invention is not particularly limited as long as it can be used for food, cosmetics and the like. For example, purified water, sterilization water, tap water, hard water, soft water, natural water, sea water, deep ocean water, electrolytic alkali ion water, electrolytic acidic ion water, ion water, cluster water and the like can be mentioned.

This water may contain preservative, isotonic agent and the like as necessary. Examples of the preservative include parabens, chlorobutanol, benzyl alcohol, propylene glycol and the like. As the isotonicity agent, glycerol, glucose, sodium chloride and the like can be mentioned.

The content of water in the assisting agent of the present invention is generally 30 - 99.999 wt%, preferably 50 - 99.99 wt%, more preferably 60 - 99.95 wt%.

In the present invention, the content of water is generally 10 - 10000 parts by weight, preferably 20 - 5000 parts by weight, more preferably 25 - 2000 parts by weight, per 1 part by weight of component (A).

While the assisting agent of the present invention can be used as it is, it may be mixed with known additives and the like as necessary and produced by a conventional method.

As the additive, water-soluble component, oily component, powder component, surfactant, polymer component, thickener, adhesiveness improver, film-forming agent, pH adjuster, antioxidizing agent, sterilizer, antimicrobial agent, preservative, firmness agent, moisturizer, skin protector, algefacient, flavor, colorant, dye, chelating agent, lubricant, anti-inflammatory agent, antipruritic agent, blood circulation promoter, astringent, tissue repair promoter, adiaphoretic, penetrant, inorganic or organic powder, ultraviolet absorber, amino acid, organic acid, inorganic salt, plant extract component, animal extract component and the like can be blended as appropriate, as long as the effect of the present invention is not inhibited.

The pH of the assisting agent of the present invention is generally pH 6.5 - 11, preferably pH 7 - 10.5, from the aspects of penetration of component (A) into the hair.

The assisting agent of the present invention may further contain a reducing agent and an alkali agent.

Examples of the reducing agent include thioglycolic acid or a salt thereof, thiolactic acid or a salt thereof, cysteine or a salt thereof, acetylcysteine or a salt thereof, cysteamine or a salt thereof, cyclic mercapto compound, sodium sulfite, glyceryl monothioglycholate and the like. Of these, thioglycolic acid or a salt thereof, cysteamine and cyclic mercapto compound are preferable, and ammonium thioglycolate and cysteamine are more preferable.

The alkali agent is not particularly limited as long as it softens and expands hair and promotes action of a reducing agent. For example, ammonia (e.g., ammonium hydroxide), alkanolamines (e.g., monoethanolamine), inorganic ammonium salts and organic ammonium salts (e.g., ammonium hydrogen carbonate, ammonium carbonate), organic amines, inorganic alkali agent, basic amino acids (e.g., arginine), alkali metal carbonates (e.g., sodium carbonate) and the like can be mentioned. Of these, ammonia and alkanolamines are preferable.

The assisting agent of the present invention containing a reducing agent and an alkali agent can be used as a first agent of a two-agent type for a permanent wave treatment or a curling treatment (hereinafter sometimes to be abbreviated as the first agent).

The first agent may be a mixture of the assisting agent of the present invention and a reducing agent and an alkali agent (single preparation). A composition containing the assisting agent of the present invention, a composition containing a reducing agent and a composition containing an alkaline agent may be separately prepared and mixed just before use to give the first agent (preparation when in use containing separate preparations).

In the above-mentioned first agent, the ratio of the combination of the assisting agent of the present invention and the reducing agent varies depending on the kind of the reducing agent. Generally, 0.1 - 100 parts by weight, preferably

0.2 - 60 parts by weight, more preferably 0.5 - 50 parts by weight, of the reducing agent is used per 1 part by weight of component (A), whether they form a single preparation or separate preparations.

In the above-mentioned first agent, the ratio of the combination of the assisting agent of the present invention and the alkali agent varies depending on the kind of the alkali agent. Generally, 0.01 - 10 parts by weight, preferably 0.02 - 8 parts by weight, more preferably 0.05 - 5 parts by weight, of the alkali agent is used per 1 part by weight of component (A), whether they form a single preparation or separate preparations.

The second agent of a two-agent type for a permanent wave treatment or a curling treatment (hereinafter sometimes to be abbreviated as the second agent) contains an oxidizing agent.

As the oxidizing agent, conventionally used ones can be employed. For example, compounds containing peroxide, such as hydrogen peroxide, sodium carbonate peroxide and urea peroxide; alkali metal peroxy salts such as sodium perborate, sodium persulfate, monopotassium persulfate; alkali metal bromate such as sodium bromate and potassium bromate, and the like can be mentioned. Of these, when the assisting agent of the present invention is used, a compound containing peroxide and alkali metal bromate are preferable, and sodium bromate and hydrogen peroxide are more preferable.

The amount of the oxidizing agent in the second agent is not particularly limited, and can be appropriately selected according to the specific object of the assisting agent of the present invention, the kind of the oxidizing agent to be used and the like.

The assisting agent of the present invention can be used as a pretreatment agent of a permanent wave treatment or a curling treatment. That is, a pretreatment agent is applied to the hair, the pretreatment agent is rinsed off as necessary, and then the first agent is applied to the hair. Generally, the first agent is applied 5 - 30 min, preferably 10 - 20 min, after application of the pretreatment agent.

The assisting agent of the present invention can be used as an intermediate agent to be used between the first agent and the second agent in a permanent wave treatment or a curling treatment. That is, the first agent is applied to the hair and then an intermediate agent is applied. To be specific, after elapse of a given time from the application of the first agent, the first agent is rinsed off as necessary, and the intermediate agent is applied. Generally, the second agent is applied 5 - 30 min, preferably 10 - 20 min, after application of the intermediate agent.

While the dosage form of the assisting agent of the present invention, first agent, pretreatment agent and intermediate agent is not particularly limited, cream, liquid, lotion, emulsion, tincture, aqueous gel agent, oily gel agent, aerosol agent, powder and the like can be mentioned, which can be produced according to a conventional method.

While the amount of the assisting agent of the present invention to be applied to the hair is appropriately adjusted according to the amount of the hair to be applied to, the length and condition thereof, and the dosage form of the assisting agent, an amount which spreads over the entire hair is generally used.

The present invention also includes a method of controlling the shape of hair, comprising the following steps:

(a) a step of applying a composition comprising component (A): a compound represented by the formula (1) or a salt thereof (hereinafter to be abbreviated as composition (1)) to hair,
(b) a step of applying a first agent for a permanent wave treatment or a curling treatment to the hair,
(c) a step of applying a second agent for a permanent wave treatment or a curling treatment to the hair.

Application to hair means coating the hair with a composition and the like or immersing the hair in a composition and the like. As a coating method and an immersion method, conventional methods can be used. The amount of the composition (1) to be applied to the hair is appropriately adjusted according to the amount of hair to be applied to, the length and condition thereof, and the dosage form of the composition, and it is usually an amount which spreads over the entire hair.

The definition of each of the amounts of other compositions, components to be contained and the like follow those in the assisting agent for hair control mentioned above.

In the method of the present invention, the order of (a) (b) (c) is not particularly limited. Generally, (a) and (b) are simultaneously performed. That is, composition (1) and the first agent are simultaneously applied to the hair, or preferably, composition (1) and the first agent are mixed in advance and applied to the hair.

In the method of the present invention, (a), (b) and (c) may be performed in this order. That is, composition (1) is applied to the hair, generally 5 - 30 min, preferably 10 - 20 min, later, the first agent is applied, and generally 5 - 30 min, preferably 10 - 20 min, later, the second agent is applied. A step of rinsing the hair with water after step (a) may also be contained.

In the method of the present invention, (b), (a) and (c) may be performed in this order. That is, the first agent is applied, generally 0 - 15 min, preferably 0 - 5 min, later, composition (1) is applied, generally 5 - 30 min, preferably 10 - 20 min, later, the second agent is applied.

In the method of the present invention, not less than 2 times of step (a) may be added. For example, (a) (b) (a) (c) may be performed in this order.

In the method of the present invention, moreover, conventionally used steps such as a washing step (step of rinsing with water etc., etc.), a drying step and the like may be further contained.

[Examples]

The present invention is explained in detail in the following by referring to Production Example and Examples. The present invention is not limited to the following Production Examples and Examples. In the present specification, "%" means "wt%" unless otherwise specified.

[Production Example 1] Synthesis of bis($N^{\varepsilon}$-lauroyl-L-lysine)sebacoyl amide disodium salt

$N^{\varepsilon}$-lauroyl-L-lysine (8.2 g, 25 mmol) was dissolved in water (70 g) and 25% aqueous sodium hydroxide solution (10 g), and diethyl ether (80 g) was added. Sebacoyl chloride (3.3 g, 14 mmol) was slowly added to the ether layer. The 2-layer solution stirred for about 1 hr while maintaining at 0°C, and stirred at room temperature for 23 hr. Then, 75% sulfuric acid was added dropwise to adjust to pH 2, and the obtained white precipitate was collected by filtration, washed well with water and dried. The obtained compound was dissolved in aqueous sodium hydroxide solution to give 10% aqueous bis($N^{\varepsilon}$-lauroyl-L-lysine)sebacoyl amide disodium salt solution.

<first agent preparation method>

At a ratio described in Table 1, a reducing agent and an alkali agent were added to water at room temperature, and the mixture was sufficiently stirred. The compound of Production Example 1 was added, and the mixture was sufficiently stirred at room temperature to give the first agent (Examples 1 - 4) (unit in Table is wt%). A mixture free of addition of the compound of Production Example 1 was used as Comparative Examples 1 and 2. The first agent used in Examples 5 and 6 was prepared at a ratio described in Table 3.

<preparation method of pretreatment agent and intermediate agent>

At a ratio described in Table 2, the compound of Production Example 1 was added to water, and the mixture was sufficiently stirred at room temperature to give a pretreatment agent (Example 5), and an intermediate agent (Example 6) (unit in Table is wt%).

<second agent preparation method>

At a ratio described in Table 4, an oxidizing agent was added to water at room temperature, and the mixture was sufficiently stirred and adjusted to pH 5 with citric acid to give a second agent (unit in Table is wt%).

<test hair>

A bundle of human hair (length 30 cm, mass 10 g) was subjected to a bleaching treatment 4 times to previously give damage, and evaluated.

<permanent wave treatment, curling treatment method>

(a) Examples 1 - 4 and Comparative Examples 1 - 2

Test hair was wetted with water, wound using paper and rod (diameter 1.5 cm), and immersed in the first agent at 35°C for 15 min. The hair was lightly washed with water, and immersed in the second agent at room temperature for 5 min. The hair was lightly washed with water, and the rod was removed without disturbing the wave. Then, the hair was immersed in 0.2 wt% aqueous SLES solution at room temperature for 30 min, sufficiently washed with water, and air dried overnight.

(b) Example 5

Test hair was wetted with water, wound using paper and rod (diameter 1.5 cm), immersed in the pretreatment agent in Table 2 for 5 min, lightly rinsed, and immersed in the first agent having the composition of Table 3 at 35°C for 15 min. The hair was treated similarly thereafter as in (a).

(c) Example 6

Test hair was wetted with water, wound using paper and rod (diameter 1.5 cm), and immersed in the first agent having the composition of Table 3 at 35°C for 15 min. Then, the hair was lightly rinsed with water, immersed in the intermediate agent in Table 2 for 5 min, lightly rinsed and similarly treated with the second agent as in (a).

Experimental Example 1 Tensile strength test of hair in water

(1) test method

apparatus used: katotech KES-G1
measurement condition: load cell 1 kg, SENS 5, speed 0.33 mm/sec
measurement method: A hair fragment (4 cm length from root) for a tensile strength test is prepared, and the major axis and the minor axis of the hair fragment are measured by laser micrometer LS-7010 MR (Keyence). The produced hair fragment is immersed in ion exchange water not less than 30 min before the start of the measurement to swell the hair fragment with water. The sample is set on a tensile strength test apparatus filled with water, the hair fragment is pulled under the above-mentioned conditions, and the 25% tensile stress (standardized at 2ϕ=80 mm) is measured. Thereafter, tensile strength per given sectional area is determined. The evaluation followed the evaluation criteria below.
⊙: tensile strength not less than 16.5 g
○: tensile strength not less than 15 g and less than 16.5 g
Δ: tensile strength more than 13 g and less than 15 g
×: tensile strength not more than 13 g

By measuring the hair tensile strength, the level of hair damage can be measured, in which an index is that a higher numerical value means less damage of hair. That is, a high numerical value of the tensile strength after a permanent wave treatment shows reduction of hair damage.

(2) moist feeling and softness of hair end

According to the following criteria, five panelists marked the moist feeling and softness of the tip of the test hair subjected to a permanent wave treatment and a curling treatment. The marks were averaged and evaluated according to the following evaluation criteria.

5 points: moist feeling of hair tip is very high and the whole hair is soft
4 points: moist feeling of hair tip is high and the whole hair is soft
3 points: normal
2 points: hair tip is rather dry, and the whole hair is stiff and not soft
1 point: hair tip is dry and the whole hair is stiff

(evaluation criteria)

⊙: average point is not less than 4.0
○: average point is not less than 3.0 and less than 4.0
Δ: average point is not less than 2.0 and less than 3.0
×: average point is less than 2.0

Table 1

| | | Ex. 1 | Ex. 2 | Ex. 3 | Ex. 4 | Comp. Ex. 1 | Comp. Ex. 2 |
|---|---|---|---|---|---|---|---|
| | compound of Prod. Ex. 1 (10% aqueous solution) | 1.0 | 5.0 | 1.0 | 5.0 | - | - |

(continued)

| | | Ex. 1 | Ex. 2 | Ex. 3 | Ex. 4 | Comp. Ex. 1 | Comp. Ex. 2 |
|---|---|---|---|---|---|---|---|
| reducing agent | 50% ammonium thioglycolate | 12.0 | 12.0 | - | - | 12.0 | - |
| | Cysteamine | - | - | 4.2 | 4.2 | - | 4.2 |
| alkali agent | 28% ammonium hydroxide | 0.5 | 0.5 | 0.5 | 0.5 | 0.5 | 0.5 |
| | 2-aminoethanol | 1.0 | 1.0 | 1.0 | 1.0 | 1.0 | 1.0 |
| | water | balance | balance | balance | balance | balance | balance |
| total | | 100.0 | 100.0 | 100.0 | 100.0 | 100.0 | 100.0 |
| result | hair tensile strength | ○ | ⊙ | ○ | ⊙ | × | Δ |
| | moist feeling and softness of hair tip | ⊙ | ⊙ | ○ | ⊙ | × | × |

Table 2

| | |
|---|---|
| composition of pretreatment agent or intermediate agent | |
| compound of Production Example 1 (10% aqueous solution) | 5 |
| water | 95 |
| total | 100 |

Table 3

| | | Ex. 5 | Ex. 6 |
|---|---|---|---|
| reducing agent | 50% ammonium thioglycolate | 12.0 | 12.0 |
| alkali agent | 28% ammonium hydroxide | 0.5 | 0.5 |
| | 2-aminoethanol | 1.0 | 1.0 |
| | water | balance | balance |
| total | | 100.0 | 100.0 |
| result | hair tensile strength | ⊙ | ⊙ |
| | moist feeling and softness of hair tip | ⊙ | ⊙ |

The formulation of the second agent used in Examples and Comparative Examples was as follows.

[Table 4]

| | | |
|---|---|---|
| oxidizing agent | sodium bromate | 7 |
| | purified water | 93 |
| | citric acid | adjusted to pH 5 |
| total | | 100 |

The results are shown in Tables 1 and 3, and Fig. 1. It was confirmed that the compound of Production Example 1 can reduce damage to the hair in a dose-dependent manner, and can provide a moist feeling and softness of the tip of hair.

Experimental Example 2 Waving efficiency and wave maintenance rate test

(1) waving efficiency

The waving efficiency after a permanent wave treatment was calculated by the Kirby method. As the hair, healthy hair (untreated hair) was used.

* Explanation on Kirby method

Twenty hairs with a length of 15 cm to 20 cm are set in one bundle, the bundle is set zigzag on an apparatus shown below and fixed at the both ends (I).

A general permanent wave treatment is applied, the hair bundle is gently removed from the apparatus, and the distance corresponding to the 5 waves thus formed is measured (II).

The waving efficiency is calculated from the following equation.

$$\text{waving efficiency } (\%) = 100 - \frac{100 \times (b-a)}{(c-a)}$$

a: distance (mm) from first rod to sixth rod of apparatus
b: distance (mm) corresponding to 5 waves formed
c: distance (mm) of b when stretched in straight line

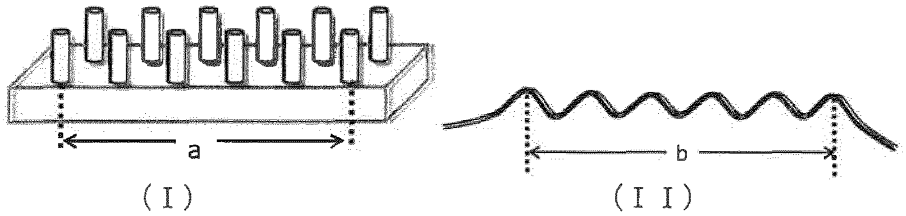


(2) wave maintenance rate

A stress test was performed at 20% SDS, 60°C for 1 hr, and the waving efficiency was calculated. The wave maintenance rate was calculated from the waving efficiency before and after the stress test, and evaluated according to the following evaluation criteria. The sustainability of the perm can be evaluated based on the calculated wave maintenance rate.

⊙: wave maintenance rate not less than 80%
○: wave maintenance rate more than 75% and less than 80%
×: wave maintenance rate not more than 75%

[Table 5]

| | | Example 3 | Comparative Example 2 |
|---|---|---|---|
| result | wave maintenance rate | ⊙ | × |

As shown in Table 5 and Fig. 2, it was confirmed that the addition of the compound of Production Example 1 to the first agent affords an effect of keeping the permed hair for a long time without changing the waving efficiency.

[INDUSTRIAL APPLICABILITY]

The present invention can provide an agent for assisting control of hair shape, which affords a superior feeling of hair touch (softness, smoothness, gathering property, gloss etc.), and a superior effect of suppressing damage to the

hair, assisting desired formation of wave shape and the like, and sustaining the shape of the hair.

## Claims

1. Use of an agent in a permanent wave treatment or a curling treatment of hair, the agent comprising component (A): a compound represented by the formula (1)

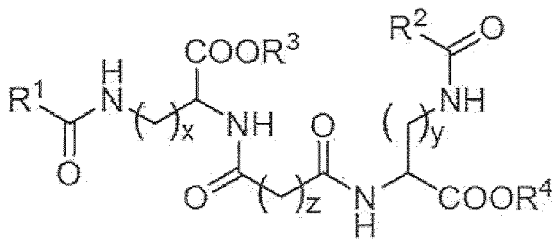

(1)

wherein

R1 and R2 are each independently an alkyl group having 5 - 21 carbon atoms or an alkenyl group having 5 - 21 carbon atoms,
R3 and R4 are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof.

2. The use according to claim 1, wherein the compound represented by the formula (1) is used for improving feeling of hair touch after a permanent wave treatment or curling treatment.

3. The use according to claim 1, wherein the compound represented by the formula (1) is used for imparting and sustaining shape of the hair whilst suppressing hair damage.

4. The use according to claim 1, wherein the compound represented by the formula (1) is used for assisting formation and holding of wave during hair wave shape adjustment.

5. The use according to any one of claims 1 to 4, wherein the component (A) is a compound of the aforementioned formula (1) wherein z is an integer of 0 - 10 or a salt thereof.

6. The use according to any one of claims 1 to 5, wherein the component (A) is a compound of the aforementioned formula (1) wherein z is 7 or 8 or a salt thereof.

7. The use according to any one of claims 1 to 6, wherein the component (A) is a compound of the aforementioned formula (1) wherein x and y are each 4 or a salt thereof.

8. The use according to any one of claims 1 to 7, wherein the component (A) is a compound of the aforementioned formula (1) wherein $R^1$ and $R^2$ are each independently a straight chain alkyl group having 5 - 15 carbon atoms or a salt thereof.

9. The use according to any one of claims 1 to 8, wherein the component (A) is a compound of the aforementioned formula (1) wherein $R^3$ and $R^4$ are each a hydrogen atom or a salt thereof.

10. The use according to any one of claims 1 to 9, wherein the component (A) is bis($N^{\varepsilon}$-lauroyl-L-lysine)sebacoyl amide or a salt thereof.

11. The use according to any one of claims 1 to 10, wherein the agent further comprises component (B): water in an amount of 10 - 10000 parts by weight per 1 part by weight of component (A).

12. A first agent for a permanent wave treatment or a curling treatment of hair, comprising:

(i) a compound represented by the formula (1)

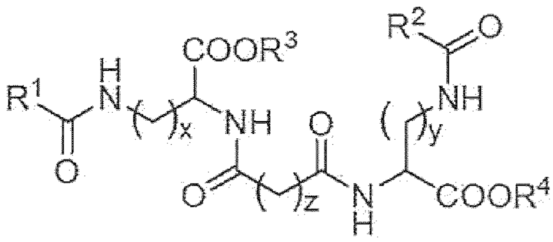

(1)

wherein

$R^1$ and $R^2$ are each independently an alkyl group having 5 - 21 carbon atoms or an alkenyl group having 5 - 21 carbon atoms,
$R^3$ and $R^4$ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof;

(ii) a reducing agent and
(iii) an alkali agent.

**13.** A method of controlling the shape of hair, comprising the following:

(a) a step of applying a composition comprising component (A): a compound represented by the formula (1)

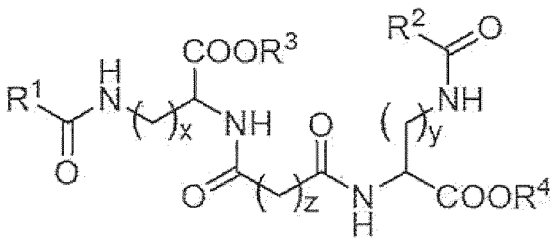

(1)

wherein

$R^1$ and $R^2$ are each independently an alkyl group having 5 - 21 carbon atoms or an alkenyl group having 5 - 21 carbon atoms,
$R^3$ and $R^4$ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof to hair,

(b) a step of applying a first agent containing a reducing agent, for a permanent wave treatment or a curling treatment to the hair,
(c) a step of applying a second agent containing an oxidizing agent, for a permanent wave treatment or a curling treatment to the hair.

**14.** The method according to claim 13, wherein (a) and (b) are simultaneously performed; or wherein (a), (b) and (c) are performed in this order; or wherein (b), (a) and (c) are performed in this order.

**Patentansprüche**

**1.** Verwendung eines Mittels bei einer Dauerwellenbehandlung oder einer Lockenbehandlung von Haar, wobei das

Mittel die Komponente (A) umfasst: eine Verbindung der Formel (1)

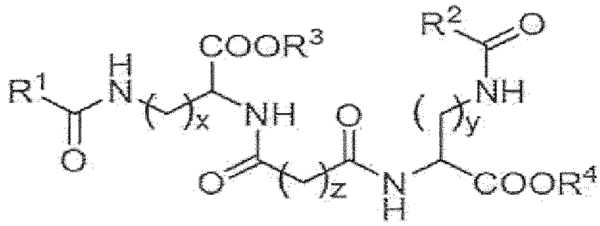

(1)

wobei

$R^1$ und $R^2$ jeweils unabhängig voneinander eine Alkylgruppe mit 5-21 Kohlenstoffatomen oder eine Alkenylgruppe mit 5-21 Kohlenstoffatomen sind,
$R^3$ und $R^4$ jeweils unabhängig voneinander ein Wasserstoffatom, eine Alkylgruppe mit 1 - 22 Kohlenstoffatomen oder eine Alkenylgruppe mit 2 - 22 Kohlenstoffatomen sind,
z eine ganze Zahl von nicht weniger als 0 ist und
x und y jeweils unabhängig voneinander eine ganze Zahl von 2 - 4 sind, oder ein Salz davon.

**2.** Verwendung nach Anspruch 1, wobei die Verbindung der Formel (1) zur Verbesserung des Gefühls der Haarberührung nach einer Dauerwellenbehandlung oder Lockenbehandlung verwendet wird.

**3.** Verwendung nach Anspruch 1, wobei die Verbindung der Formel (1) zum Verleihen und Erhalten der Form des Haares bei gleichzeitiger Unterdrückung von Haarschäden verwendet wird.

**4.** Verwendung nach Anspruch 1, wobei die Verbindung der Formel (1) zur Unterstützung der Bildung und des Haltens der Welle während der Anpassung der Haarwellenform verwendet wird.

**5.** Verwendung nach einem der Ansprüche 1 bis 4, wobei die Komponente (A) eine Verbindung der vorstehend genannten Formel (1), worin z eine ganze Zahl von 0 - 10 ist, oder ein Salz davon ist.

**6.** Verwendung nach einem der Ansprüche 1 bis 5, wobei die Komponente (A) eine Verbindung der vorstehend genannten Formel (1), worin z 7 oder 8 ist, oder ein Salz davon ist.

**7.** Verwendung nach einem der Ansprüche 1 bis 6, wobei die Komponente (A) eine Verbindung der vorstehend genannten Formel (1), worin x und y jeweils 4 sind, oder ein Salz davon ist.

**8.** Verwendung nach einem der Ansprüche 1 bis 7, wobei die Komponente (A) eine Verbindung der vorstehend genannten Formel (1), worin $R^1$ und $R^2$ jeweils unabhängig voneinander eine geradkettige Alkylgruppe mit 5 - 15 Kohlenstoffatomen sind, oder ein Salz davon ist.

**9.** Verwendung nach einem der Ansprüche 1 bis 8, wobei die Komponente (A) eine Verbindung der vorstehend genannten Formel (1), worin $R^3$ und $R^4$ jeweils ein Wasserstoffatom sind, oder ein Salz davon ist.

**10.** Verwendung nach einem der Ansprüche 1 bis 9, wobei die Komponente (A) bis ($N^{\varepsilon}$-Lauroyl-L-lysin)sebacoylamid oder ein Salz davon ist.

**11.** Verwendung nach einem der Ansprüche 1 bis 10, wobei das Mittel ferner die Komponente (B) umfasst: Wasser in einer Menge von 10 bis 10000 Gewichtsteilen pro 1 Gewichtsteil der Komponente (A).

**12.** Erstes Mittel für eine Dauerwellenbehandlung oder eine Lockenbehandlung von Haar, umfassend:

(i) eine Verbindung der Formel (1)

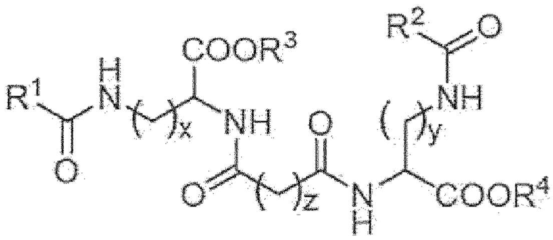

(1)

wobei

$R^1$ und $R^2$ jeweils unabhängig voneinander eine Alkylgruppe mit 5 - 21 Kohlenstoffatomen oder eine Alkenylgruppe mit 5 - 21 Kohlenstoffatomen sind,
$R^3$ und $R^4$ jeweils unabhängig voneinander ein Wasserstoffatom, eine Alkylgruppe mit 1 - 22 Kohlenstoffatomen oder eine Alkenylgruppe mit 2 - 22 Kohlenstoffatomen sind,
z eine ganze Zahl von nicht weniger als 0 ist, und
x und y jeweils unabhängig voneinander eine ganze Zahl von 2 - 4 sind, oder ein Salz davon;

(ii) ein Reduktionsmittel und
(iii) ein alkalisches Mittel.

**13.** Verfahren zur Kontrolle der Form von Haaren, umfassend das Folgende:

(a) einen Schritt, bei dem eine Zusammensetzung, die die Komponente (A) enthält: eine Verbindung der Formel (1)

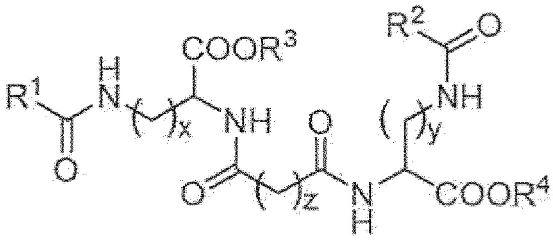

(1)

wobei

$R^1$ und $R^2$ jeweils unabhängig voneinander eine Alkylgruppe mit 5 - 21 Kohlenstoffatomen oder eine Alkenylgruppe mit 5 - 21 Kohlenstoffatomen sind,
$R^3$ und $R^4$ sind jeweils unabhängig voneinander ein Wasserstoffatom, eine Alkylgruppe mit 1 - 22 Kohlenstoffatomen oder eine Alkenylgruppe mit 2 - 22 Kohlenstoffatomen sind,
z eine ganze Zahl von nicht weniger als 0 ist, und
x und y jeweils unabhängig voneinander eine ganze Zahl von 2 - 4 sind, oder ein Salz davon auf Haar aufgetragen wird,

(b) einen Schritt, bei dem ein erstes Mittel, das ein Reduktionsmittel enthält, für eine Dauerwellenbehandlung oder eine Lockenbehandlung auf das Haar aufgetragen wird,
(c) einen Schritt, bei dem ein zweites Mittel, das ein Oxidationsmittel enthält, für eine Dauerwellenbehandlung oder eine Lockenbehandlung auf das Haar aufgetragen wird.

**14.** Verfahren nach Anspruch 13, wobei (a) und (b) gleichzeitig ausgeführt werden; oder
wobei (a), (b) und (c) in dieser Reihenfolge ausgeführt werden; oder
wobei (b), (a) und (c) in dieser Reihenfolge ausgeführt werden.

**Revendications**

**1.** Utilisation d'un agent dans un traitement de permanente ou un traitement de frisage des cheveux, l'agent comprenant

un composant (A) : un composé représenté par la formule (1)

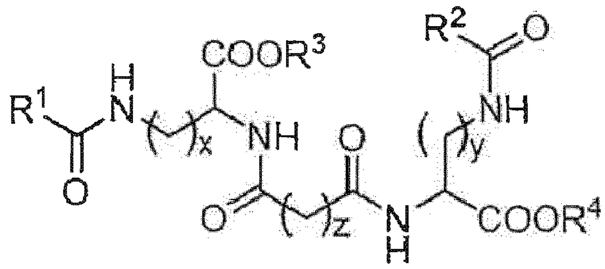

(1)

dans laquelle

chacun de $R^1$ et $R^2$ est indépendamment un groupe alkyle ayant 5 à 21 atomes de carbone ou un groupe alcényle ayant 5 à 21 atomes de carbone,
chacun de $R^3$ et $R^4$ représente indépendamment un atome d'hydrogène, un groupe alkyle ayant 1 à 22 atomes de carbone ou un groupe alcényle ayant 2 à 22 atomes de carbone,
z est un entier non inférieur à 0, et
chacun de x et y est indépendamment un entier de 2 à 4,
ou un sel de celui-ci.

**2.** Utilisation selon la revendication 1, dans laquelle le composé représenté par la formule (1) est utilisé pour améliorer la sensation au toucher des cheveux après un traitement de permanente ou un traitement de frisage.

**3.** Utilisation selon la revendication 1, dans laquelle le composé représenté par la formule (1) est utilisé pour conférer un maintien de forme aux cheveux tout en supprimant les dommages aux cheveux.

**4.** Utilisation selon la revendication 1, dans laquelle le composé représenté par la formule (1) est utilisé pour faciliter la formation et le maintien d'une ondulation durant un ajustement de forme ondulée des cheveux.

**5.** Utilisation selon l'une quelconque des revendications 1 à 4, dans laquelle le composant (A) est un composé de formule (1) susmentionnée dans laquelle z est un entier de 0 à 10 ou un sel de celui-ci.

**6.** Utilisation selon l'une quelconque des revendications 1 à 5, dans laquelle le composant (A) est un composé de formule (1) susmentionnée dans laquelle z vaut 7 ou 8 ou un sel de celui-ci.

**7.** Utilisation selon l'une quelconque des revendications 1 à 6, dans laquelle le composant (A) est un composé de formule (1) susmentionnée dans laquelle chacun de x et y vaut 4 ou un sel de celui-ci.

**8.** Utilisation selon l'une quelconque des revendications 1 à 7, dans laquelle le composant (A) est un composé de formule (1) susmentionnée dans laquelle chacun de $R^1$ et $R^2$ est indépendamment un groupe alkyle à chaîne droite ayant 5 à 15 atomes de carbone ou un sel de celui-ci.

**9.** Utilisation selon l'une quelconque des revendications 1 à 8, dans laquelle le composant (A) est un composé de formule (1) susmentionnée dans laquelle chacun de $R^3$ et $R^4$ est un atome d'hydrogène ou un sel de celui-ci.

**10.** Utilisation selon l'une quelconque des revendications 1 à 9, dans laquelle le composant (A) est le bis($N^{\varepsilon}$-lauroyl-L-lysine)sébaçoyl-amide ou un sel de celui-ci.

**11.** Utilisation selon l'une quelconque des revendications 1 à 10, dans laquelle l'agent comprend en outre un composant (B) : de l'eau en une quantité de 10 à 10 000 parties en poids pour 1 partie en poids du composant (A).

**12.** Premier agent pour un traitement de permanente ou un traitement de frisage des cheveux, comprenant :

(i) un composé représenté par la formule (1)

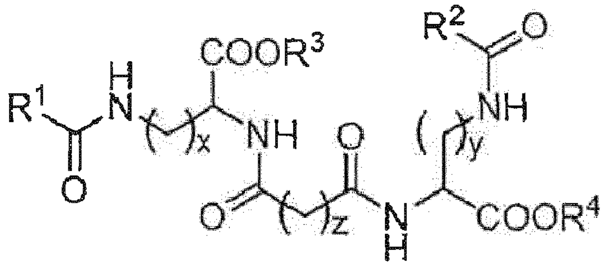

(1)

dans laquelle

chacun de $R^1$ et $R^2$ est indépendamment un groupe alkyle ayant 5 à 21 atomes de carbone ou un groupe alcényle ayant 5 à 21 atomes de carbone,
chacun de $R^3$ et $R^4$ représente indépendamment un atome d'hydrogène, un groupe alkyle ayant 1 à 22 atomes de carbone ou un groupe alcényle ayant 2 à 22 atomes de carbone,
z est un entier non inférieur à 0, et
chacun de x et y est indépendamment un entier de 2 à 4,
ou un sel de celui-ci ;

(ii) un agent réducteur et
(iii) un agent alcalin.

**13.** Procédé pour contrôler la forme des cheveux, comprenant ce qui suit :

(a) une étape d'application sur les cheveux d'une composition comprenant un composant (A) : un composé représenté par la formule (1)

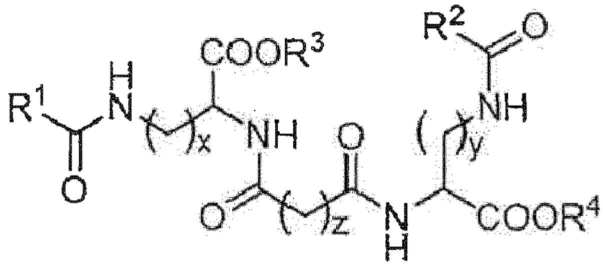

(1)

dans laquelle

chacun de $R^1$ et $R^2$ est indépendamment un groupe alkyle ayant 5 à 21 atomes de carbone ou un groupe alcényle ayant 5 à 21 atomes de carbone,
chacun de $R^3$ et $R^4$ représente indépendamment un atome d'hydrogène, un groupe alkyle ayant 1 à 22 atomes de carbone ou un groupe alcényle ayant 2 à 22 atomes de carbone,
z est un entier non inférieur à 0, et
chacun de x et y est indépendamment un entier de 2 à 4,
ou un sel de celui-ci,

(b) une étape d'application sur les cheveux d'un premier agent contenant un agent réducteur, pour un traitement de permanente ou un traitement de frisage,
(c) une étape d'application sur les cheveux d'un deuxième agent contenant un agent oxydant, pour un traitement de permanente ou un traitement de frisage.

**14.** Procédé selon la revendication 13, dans lequel les étapes (a) et (b) sont effectuées simultanément ; ou
dans lequel les étapes (a), (b) et (c) sont effectuées dans cet ordre ; ou
dans lequel les étapes (b), (a) et (c) sont effectuées dans cet ordre.

Fig. 1

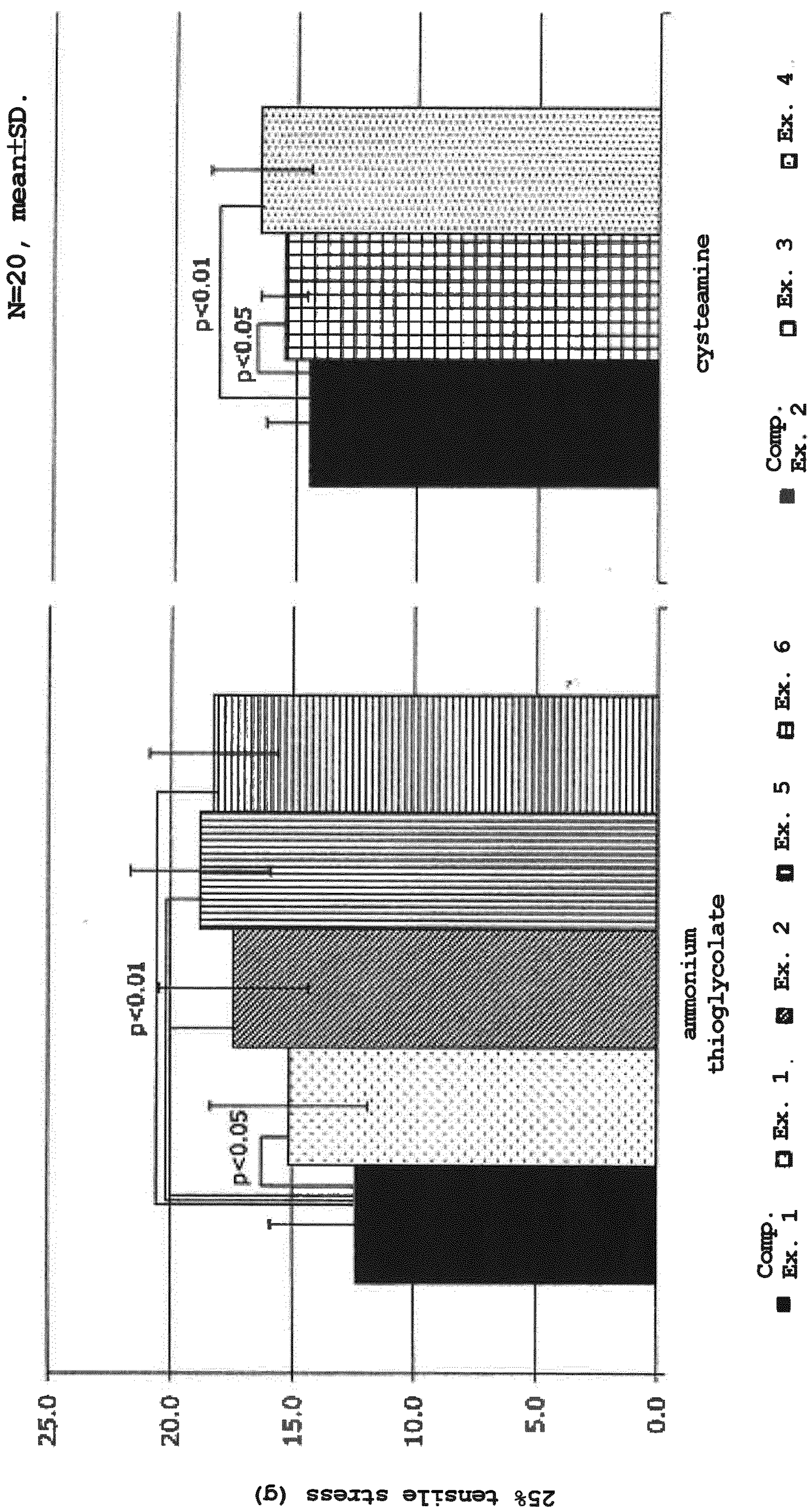
N=20, mean±SD.
25% tensile stress (g)
25.0
20.0
15.0
10.0
5.0
0.0
p<0.05
p<0.01
ammonium thioglycolate
cysteamine
Comp. Ex. 1
Ex. 1
Ex. 2
Ex. 5
Ex. 6
Comp. Ex. 2
Ex. 3
Ex. 4

Fig. 2

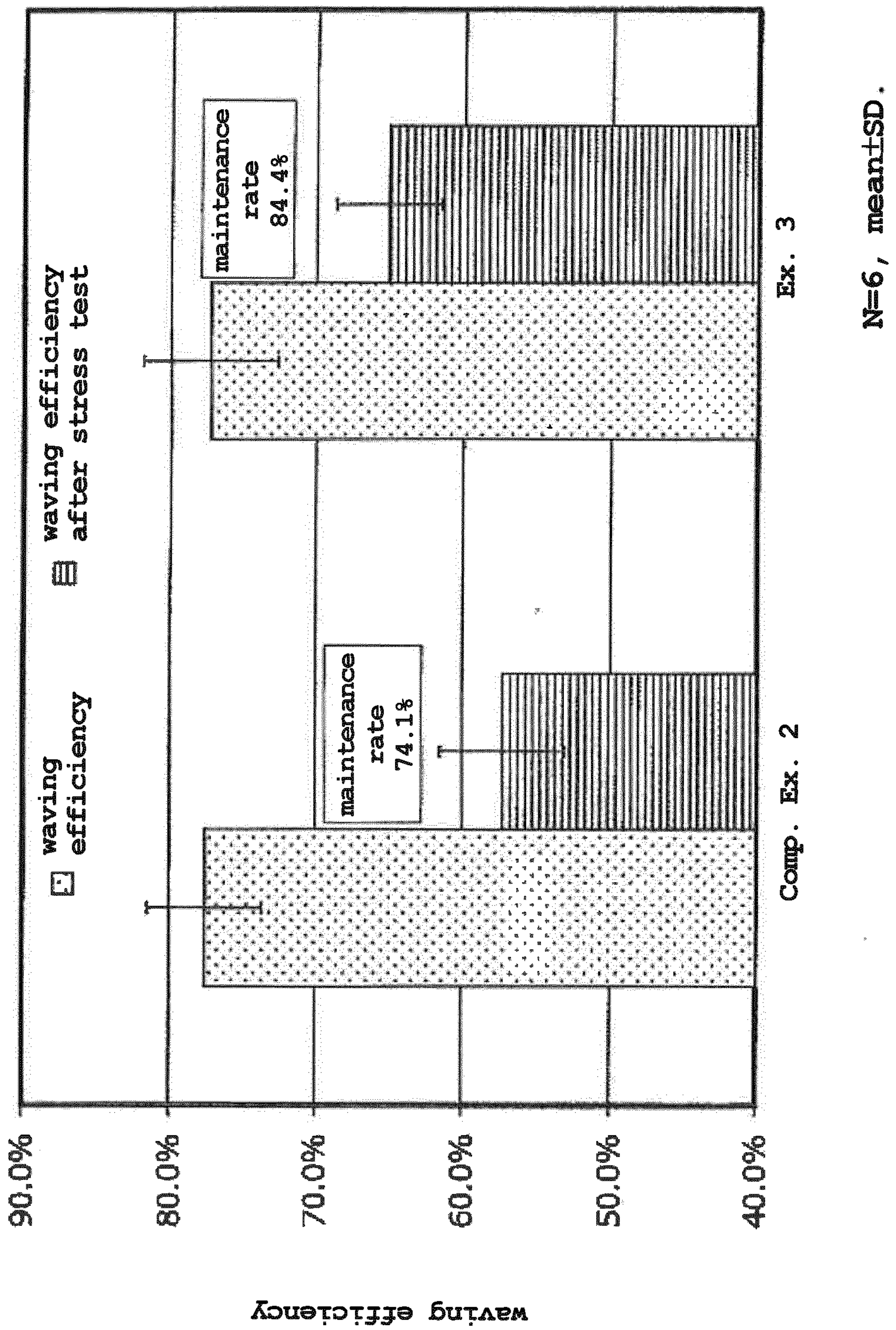
waving efficiency
waving efficiency after stress test
maintenance rate 74.1%
maintenance rate 84.4%
90.0%
80.0%
70.0%
60.0%
50.0%
40.0%
waving efficiency
Comp. Ex. 2
Ex. 3
N=6, mean±SD.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007001916 A
- JP 2009057335 A
- JP 2013095696 A
- JP 2004323505 A


### Non-patent literature cited in the description


- *Org. Biomol. Chem.,* 2003, vol. 1, 4124-4131
- *New J. Chem.,* 2005, vol. 29, 1439-1444